# EUROPEAN PATENT APPLICATION

(11) **EP 3 446 728 A1**
(43) Date of publication of application: **27.02.2019**
(21) Application number: 16899397.0
(22) Date of filing: 20.04.2016
(51) Int. Cl.: A61M 1/10

(54) **BLOOD PUMP**

(71) Applicant: Sun Medical Technology Research Corporation, Suwa-shi, Nagano 392-0012 (JP)
(72) Inventor: MIYAKOSHI, Takayuki, Suwa-shi Nagano 392-0012 (JP)
(74) Representative: Liesegang, Eva
(86) International application number: PCT/JP2016/062456
(87) International publication number: WO 2017/183124

(57) **Abstract**

A blood pump (100) according to the present invention includes: a base body (110) ; a casing (120) fitted on the base body (110); a blood supply mechanism housed in a pump chamber surrounded by the base body and the casing; and a drive element supplying energy to the blood supply mechanism. The base body (110) has: an approximately horizontal surface (111) including a first contact surface (113) which is brought into contact with the casing (120); and a first engaging portion (115) including a first tapered portion (116) which is inclined inward as the first tapered portion (116) extends downward. The casing (120) has: a casing body (121) having a second contact surface (122) at a position which corresponds to the first contact surface (113); and a second engaging portion (126) formed at an edge side of the casing (120) and including a second tapered portion (126) which is inclined toward an r direction as the second tapered portion (126) extends upward from an inner end portion (127). The second engaging portion engages with the first engaging portion, and the second contact surface of the casing is pressed so as to be brought into contact with the first contact surface of the base body. The formation of a gap between the second contact surface and the first contact surface is prevented. It is also possible to provide a blood pump having a small diameter and a small volume.

## Description

### Technical Field

The present invention relates to a blood pump.

### Background Art

There has been known a blood pump which is used in an auxiliary artificial heart or the like (see patent literature 1, for example). The blood pump is provided for assisting a blood supply function of the user's heart. The blood pump has, as a basic function thereof, a blood supply function for supplying blood of the user into the inside of a body of the user by allowing the blood of the user to flow into a pump chamber and to flow out from the pump chamber. In the inside of the pump chamber of the blood pump, it is necessary to house a blood supply mechanism such as an impeller which forms a movable part. Accordingly, in general, in manufacturing the blood pump, a base body and a casing are prepared as separate parts respectively, and a blood supply mechanism is housed in a pump chamber surrounded by the base body and the casing and, thereafter, the blood pump is completed by combining the base body and the casing together.

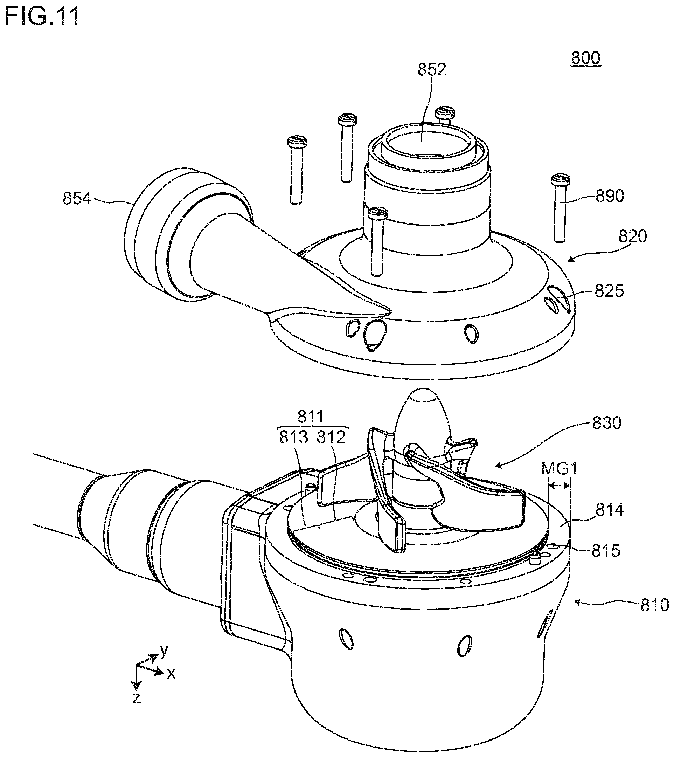

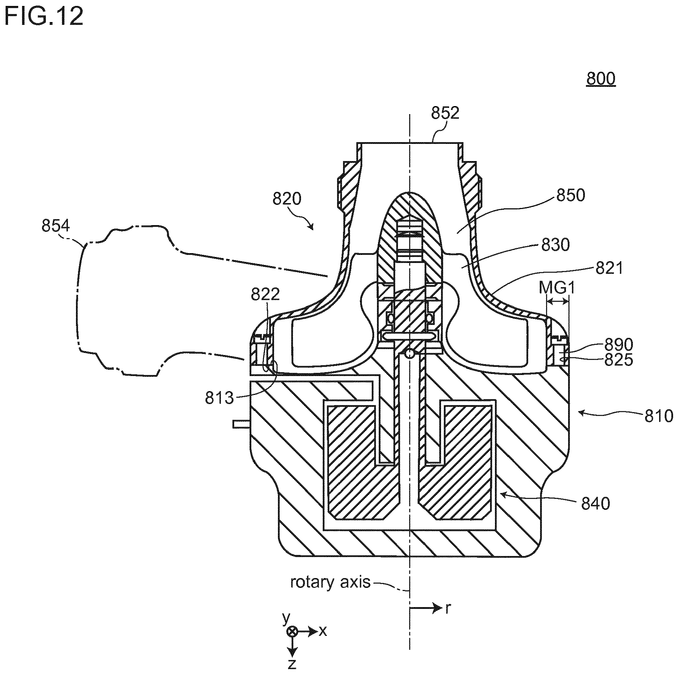

Fig. 11 and Fig. 12 are views for describing a conventional blood pump 800. Fig. 11 is a perspective view showing a state before a casing 820 is joined to a base body 810. Fig. 12 is a cross-sectional view showing a state after the casing 820 is joined to the base body 810.

As shown in Fig. 11 and Fig. 12, the conventional blood pump 800 includes: the base body 810; the casing 820 fitted on the base body 810; a blood supply mechanism 830 housed in a pump chamber 850 surrounded by the base body 810 and the casing 820; and a drive element 840 mounted on the base body 810 and supplying energy to the blood supply mechanism 830. In such a blood pump 800, the blood supply mechanism 830 allows blood to flow into the pump chamber 850 and to flow out from the pump chamber 850, and supplies blood into the inside of a body of a user (not shown in the drawing) . In the description of the conventional blood pump 800, assuming a direction that the casing 820 is fitted on the base body 810 by sliding as a z direction, a direction perpendicular to the z direction as an x direction, a direction perpendicular to the z direction and the x direction respectively as a y direction, and a direction directed from a center portion of the base body 810 to the outside of the base body 810 when an xy plane is viewed in a plan view along the z direction as an r direction, the base body 810 has: a blood contact surface 812 which faces the pump chamber 850; a first contact surface 813 which is brought into contact with the casing 820; and a screw fastening margin 814 formed on a more r direction side than the first contact surface 813; and female screws 815 formed in a region of the screw fastening margin 814 in a state where the female screws 815 extend in the z direction, and the casing 820 has: a second contact surface 822 (not shown in Fig. 11) formed on the casing 820 at a position corresponding to the first contact surface 813; and play holes 825 formed on an edge side of the casing 820. Then, in the blood pump 800, by allowing male screws 890 to pass through the play holes 825 formed in the casing 820 and to threadedly engage with the female screws 815 of the base body 810, the base body 810 and the casing 820 are combined together and are threaded with each other, and the second contact surface 822 of the casing 820 is pressed so as to be brought into contact with the first contact surface 813 of the base body 810.

In the conventional blood pump 800, (1) the base body 810 and the casing 820 are formed as separate parts from each other and hence, the blood supply mechanism 830 which forms the movable part can be housed in the pump chamber 850, and (2) the male screws 890 pass through the play holes 825 formed in the casing 820 and threadedly engage with the female screws 815 of the base body 810 and hence, the base body 810 and the casing 820 are combined together and are threaded with each other, and the second contact surface 822 of the casing 820 is pressed so as to be brought into contact with the first contact surface 813 of the base body 810 and hence, even when pressure in the pump chamber 850 is increased, there is no possibility that a gap is formed between the base body 810 and the casing 820 (between the first contact surface 813 and the second contact surface 822).

### Citation List

### Patent Literature

PTL 1: JP-A-2009-297174
PTL 2: JP-A-2006-258720

### Summary of Invention

### Technical Problem

However, in the conventional blood pump 800, to ensure the above-mentioned pressing force for pressing the second contact surface 822 to the first contact surface 813, it is necessary to adopt the screws (male screws 890 and female screws 815) having a relatively large diameter. Accordingly, it is necessary to ensure a relatively large width of the screw fastening margin 814 (the width being indicated by MG1) in Fig. 11 and Fig. 12). As a result, it is unavoidable that a diameter, a volume and the like of the whole blood pump become relatively large.

However, a thickness of a chest of a person is limited and hence, it is extremely important that a diameter, a volume and the like of the blood pump are set as small as possible. When the blood pump is small, it is possible to embed the blood pump into the inside of the body of a person (patient) having a small physical build such as a child, for example and hence, the number of people who can use a blood pump can be increased. In this manner, there has been a strong demand for the miniaturization of a blood pump in a medical field on the other hand.

To satisfy such a demand, while passing on the adoption of screws which require the screw fastening margin 814 having a relatively large width (MG1), it is considered preferable to introduce the back cover mounting structure of a watch (background art: see patent literature 2, for example) which is popularly used in general into a blood pump.

The back cover mounting structure 900 which forms a background art has the structure where a base body (back cover) 910 is mounted on a casing (watch case) 920 by pushing a first engaging portion (dowel portion) 915 of the base body (back cover) 910 into a second engaging portion (fitting portion) 925 of the casing (watch case) 920. In such a configuration, the first engaging portion (dowel portion) 915 generates an elastic force and acts on a second engaging convex portion 927, and presses the second engaging convex portion 927 such that a casing lower end portion (end surface) 929 is brought into contact with an edge portion of a back cover (edge portion) 914 (see Fig. 13 and paragraph [0012] of patent literature 2 and the like).

However, the back cover mounting structure 900 of the watch which forms a background art has at least the following drawbacks (1) to (3).
(1) The first engaging portion (dowel portion) 915 is formed using a super elastic material, has a shape formed by bending a thin plate material, and is soft. Further, as a structure considered as a premise in the field of watches, the back cover mounting structure which adopts such fitting engagement is, in general, configured such that "slits" are formed in the structure along an outer periphery of the structure (the back cover mounting structure not being formed of a continuous ring but being a ring having an outer periphery on which a plurality of slits are partially formed along the outer periphery). Due to such a configuration, it is safe to say that the first engaging portion (dowel portion) 915 is a member such as an extremely soft spring. However, with the such a structure which uses the first engaging portion (dowel portion) 915, it is difficult to generate a sufficient pressing force from a lower end portion (end surface) 929 of the casing to the edge portion of the back cover (edge portion) 914.
(2) A packing 960 is pressed and collapsed in a z direction (lower direction) by the second engaging convex portion 927 so that a repulsive force from the packing 960 in a -z direction (direction opposite to the z direction, an upward direction in the drawing) decreases the above-mentioned pressing force to the contrary.
(3) The packing 960 is pressed and collapsed so that an area of the packing 960 on an xy plane is expanded. Accordingly, a diameter of the back cover mounting structure (blood pump when the back cover structure is applied to the blood pump, for example) in an r direction is increased by an expansion amount.

In the above-mentioned back cover mounting structure, assume a direction that the casing (watch case) 920 is fitted on the base body (back cover) 910 by sliding as a z direction, a direction perpendicular to the z direction as an x direction, a direction perpendicular to the z direction and the x direction respectively as a y direction, and a direction directed from a center portion of the base body 810 to the outside of the base body 810 when an xy plane is viewed in a plan view along the z direction as an r direction.

In this manner, the back cover mounting structure 900 of the watch which forms a background art has the above-mentioned drawbacks and hence, the back cover mounting structure 900 cannot be adopted by the blood pump.

For a reference purpose, one required specification of the blood pump is described. That is, as one of required specifications of the blood pump, there is a specification that even when a pressure in a pump chamber on a blood side is increased, the formation of a gap between a contact surface on a casing side which defines the pump chamber and a contact surface on a base body side which defines the pump chamber must be prevented by all means . Assuming a case where a gap is formed between the contact surfaces, due to the formation of such a gap, a slight amount of blood which intrudes into the gap is stagnated and is solidified. There is a possibility that this solidified blood clot (thrombus) is peeled off from the gap due to agitation in the pump chamber by a blood supply mechanism such as an impeller and returns to the pump chamber again. Depending on a case, there is also a possibility that such a blood clot flows out into the inside of a body of a user (patient) and causes an undesirable condition.

Due to such a reason, the formation of the gap between the contact surfaces must be prevented by all means. Even when a pressure in the pump chamber is increased, to prevent the contact surface on the casing side from floating from the contact surface on the base body side, it is necessary to constantly apply a large pressing force from the contact surface on the casing side to the contact surface on the base body side.

On the other hand, in the case of a watch, there is no possibility that a liquid flows in the watch and no high pressure is generated in the watch. Accordingly, a force which intends to remove the casing from the base body is not generated in the casing. Further, the watch must satisfy a fundamental required specification that, even after the base body and the casing are engaged with each other by fitting engagement, the casing can be repeatedly fitted on or detached from the base body for repair or the like.

In the blood pump, the generation of a high pressure from the inside is estimated as a premise as described previously. Under such circumstances, the removal of the casing from the base body must be absolutely prevented, and the formation of a gap at a contact surface where the base body and the casing are brought into contact with each other and which defines a pump chamber must be prevented by all means.

In this manner, the required specification of the watch and the required specification of the blood pump are completely opposite from each other. Since the watch and the blood pump differ from each other in required specification, the watch and the blood pump also differ in a design concept, configuration, and functions which the configuration exhibits. Accordingly, even when the back cover mounting structure 900 of the watch which is proposed based on the different concept is introduced to the blood pump, such a structure cannot satisfy the specification required by the blood pump. Accordingly, there has been a demand for zero-based development of the structure of the blood pump.

The present invention has been made in view of the above-mentioned circumstances, and it is an object of the present invention to provide a blood pump which can constantly apply a large pressing force from a contact surface on the casing side to a contact surface on the base body side so that even when a pressure in a pump chamber is increased, no gap is formed between the contact surface on the casing side which defines the pump chamber and the contact surface on the base body side which defines the pump chamber.

It is another object of the present invention to provide a blood pump having a diameter, a volume and the like which are as small as possible.

It is still another object of the present invention to provide a blood pump which can achieve both of the above-mentioned objects.

It is a further object of the present invention to provide a blood pump which can prevent a leakage of blood to the outside, and can prevent the intrusion of a bodily fluid or the like from the outside of the blood pump into the inside of the blood pump.

### Solution to Problem

[1] A blood pump according to a first aspect of the present invention is a blood pump which includes:
   a base body;
   a casing fitted on the base body;
   a blood supply mechanism housed in a pump chamber surrounded by the base body and the casing; and
   a drive element mounted on the base body for supplying energy to the blood supply mechanism, wherein
   the blood supply mechanism is configured to allow blood to flow into the pump chamber and to flow out from the pump chamber supplying blood into the inside of a body of a user by the blood supply mechanism, wherein
   assuming a direction that the casing is fitted on the base body by sliding as a z direction, a direction perpendicular to the z direction as an x direction, a direction perpendicular to the z direction and the x direction respectively as a y direction, and a direction directed from a center portion of the base body to the outside of the base body when an xy plane is viewed in a plan view along the z direction as an r direction,
   the base body has:
   an approximately horizontal surface formed on a -z direction side, and including a blood contact surface which faces the pump chamber and a first contact surface which is brought into contact with the casing; and
   a first engaging portion formed on an r direction side and including a first tapered portion inclined toward a -r direction as the first tapered portion extends from an outer end portion of the first engaging portion in the z direction, and
   the casing has:
      a casing body having a second contact surface at a position which corresponds to the first contact surface;
      a second engaging portion formed at an edge side of the casing, and including a second tapered portion inclined toward the r direction as the second tapered portion extends from an inner end portion in the -z direction on an inner wall of the casing; and
      a casing intermediate portion positioned between the casing body and the second engaging portion, wherein
      the blood pump is configured such that the second engaging portion of the casing engages with the first engaging portion of the base body, and the second contact surface of the casing is pressed so as to be brought into contact with the first contact surface of the base body.

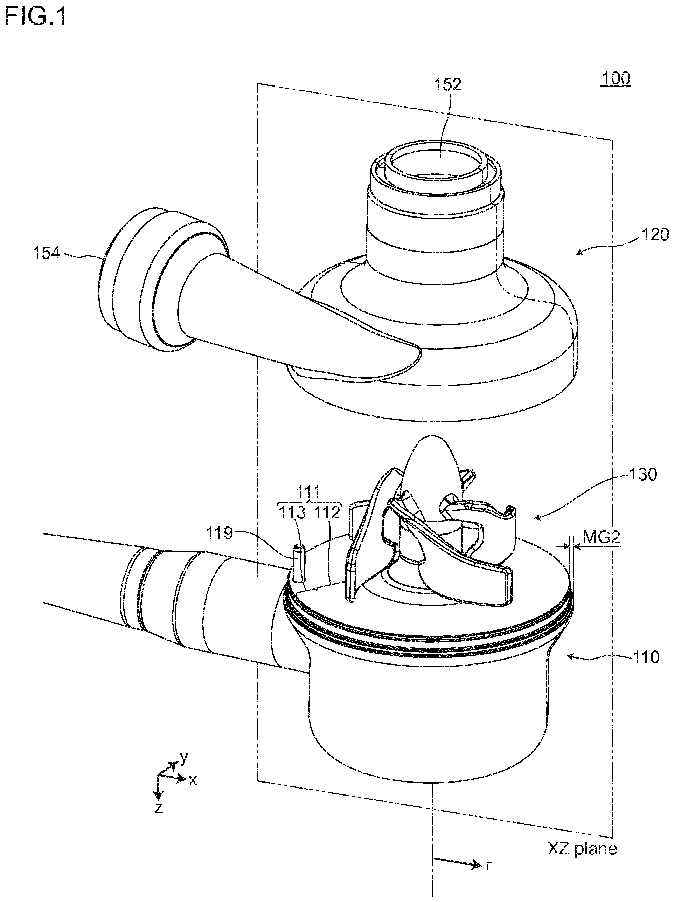

   In the blood pump according to the first aspect of the present invention, the base body having the first engaging portion which includes the first tapered portion inclined toward the -r direction as the first tapered portion extends from the outer end portion in the z direction, and the casing having the second engaging portion which is formed at the edge side of the casing and which includes the second tapered portion inclined toward the r direction as the second tapered portion extends from the inner end portion in the -z direction on the inner wall of the casing engage with each other between the second engaging portion and the first engaging portion, and the second contact surface of the casing is pressed so as to be brought into contact with the first contact surface of the base body. Accordingly, the blood pump according to the first aspect of the present invention can acquire at least the following advantageous effects (a) to (d).
   (a) A large pressing force can be constantly applied from the second contact surface on the casing side to the first contact surface on the base body side and hence, even when pressure in the pump chamber is increased, it is possible to prevent the formation of a gap between the second contact surface on the casing side which defines the pump chamber and the first contact surface on the base body side which defines the pump chamber.
   (b) By adopting the above-mentioned fitting engagement structure, it is unnecessary to use screws. Accordingly, while satisfying the above-mentioned required specification of the blood pump, portions irrelevant to a basic function (blood supply function) of the blood pump (the respective contact surfaces of the base body and the casing (MG1 and MG2) not directly forming the pump chamber unlike blood contact surfaces thus being irrelevant to the basic function) can be made as small as possible and hence, the blood pump can be formed with a margin MG1 (described later with reference to Fig. 1) which is far smaller than the screw fastening margin MG2. As a result, it is possible to provide the blood pump having a small diameter and a small volume compared to conventional blood pumps.
   (c) It is possible to provide the blood pump which can achieve both of the above-mentioned advantageous effects (a) and (b).
   (d) Due to the above-mentioned advantageous effects, as a matter of course, it is possible to provide the blood pump which can prevent a leakage of blood to the outside and can prevent the intrusion of a bodily fluid or the like from the outside of the blood pump into the inside of the blood pump.
[2] In the blood pump according to the first aspect of the present invention, it is preferable that
   assuming an inner diameter of the inner end portion of the second tapered portion of the casing as φA and an outer diameter of the outer end portion of the first tapered portion of the base body as φB before the casing is fitted on the base body, a relationship of φA<φB be established, and
   the casing be fitted on the base body in a state that the inner end portion of the second tapered portion is positioned at a portion of the first tapered portion shifted in a z direction side from the outer end portion, the portion being retracted from a terminal end portion of the first engaging portion formed on a side opposite to the outer end portion.
[3] In the blood pump according to the first aspect of the present invention, it is preferable that
   a casing lower end portion which is disposed on an edge side of the casing be not brought into contact with the base body in a direction along the z direction.
[4] In the blood pump according to the first aspect of the present invention, it is preferable that
   as viewed in a cross section of the blood pump taken along an xz plane, assuming an angle made by a profile of an inclined surface of the first tapered portion and the z direction as θ1 and an angle made by a profile of an inclined surface of the second tapered portion and the z direction as θ2, a relationship of θ1<θ2 be established.
[5] In the blood pump according to the first aspect of the present invention, it is preferable that
   as viewed in a cross section of the blood pump taken along an xz plane,
   assuming a thickness of the casing body in a direction perpendicular to a tangent plane on an outer side of the casing body as t1, a thickness of the casing intermediate portion in a direction perpendicular to a tangent plane of an outer side of the casing intermediate portion as t2, and a thickness of the second engaging portion in a direction perpendicular to a tangent plane of an outer side of the second engaging portion as t3, a relationship of t1>t2>t3 be established, and
   with respect to an inclined surface which forms the second tapered portion of the second engaging portion, at a portion on an end of the inclined surface in the -z direction which is a side opposite to the inner end portion, the casing have a smallest thickness.
[6] In the blood pump according to the first aspect of the present invention, it is preferable that, a mirror finish be applied to the approximately horizontal surface of the base body.
[7] In the blood pump according to the first aspect of the present invention, it is preferable that,
   as viewed in a plan view of the first engaging portion and the second engaging portion taken along an xy plane respectively along the z direction, the first engaging portion and the second engaging portion have a circular shape.
[8] In the blood pump according to the first aspect of the present invention, it is preferable that the casing be made of a material which contains titanium as a main component.
[9] A blood pump according to a second aspect of the present invention is preferably configured such that, in the blood pump according to the first aspect of the present invention described in [1] to [8],
   the base body have a packing groove in which a packing is disposed at a position on a more -z direction side of the first engaging portion,
   the casing have an intermediate portion inner wall at a position of the casing intermediate portion, and
   the packing be disposed such that the packing is sandwiched between the packing groove and the intermediate portion inner wall.
[10] In the blood pump according to the second aspect of the present invention, it is preferable that,
   when the blood pump is viewed in a plan view along the z direction, the packing groove formed in the base body overlap with the approximately horizontal surface of the base body, and the second contact surface of the casing overlap with at least a portion of the packing.
[11] In the blood pump according to the first or the second aspect of the present invention, it is preferable that the blood supply mechanism be an impeller, and the drive element be a motor for driving the impeller.
[12] In the blood pump according to the first or the second aspect of the present invention, it is preferable that the blood pump be used in the form of an auxiliary artificial heart by embedding the blood pump in the inside of the body.

### Brief Description of Drawings

Fig. 1 is a view for describing a blood pump 100 according to an embodiment 1.
Fig. 2 is a view for describing the blood pump 100 according to the embodiment 1.
Fig. 3(a) to Fig. 3(c) are views for describing a main part of the blood pump 100 according to the embodiment 1.
Fig. 4 is a view for describing a size relationship of the main part of the blood pump 100 according to the embodiment 1.
Fig. 5 is a view for describing a manner in which a pressing force F2 which acts from a second contact surface 122 to a first contact surface 113 is generated in the blood pump 100 according to the embodiment 1.
Fig. 6 is a view for describing an auxiliary artificial heart system 300 where the blood pump 100 according to the embodiment 1 is embedded into a body.
Fig. 7 is a view for describing a main part of a blood pump 100a according to an embodiment 2.
Fig. 8 is a view for describing a blood pump 100b according to an embodiment 3.
Fig. 9 is a view for describing a result of evaluation of a blood pump according to an example.
Fig. 10 is a view for describing a main part of a blood pump according to a modification 2.
Fig. 11 is a view for describing a conventional blood pump 800.
Fig. 12 is a view for describing the conventional blood pump 800.

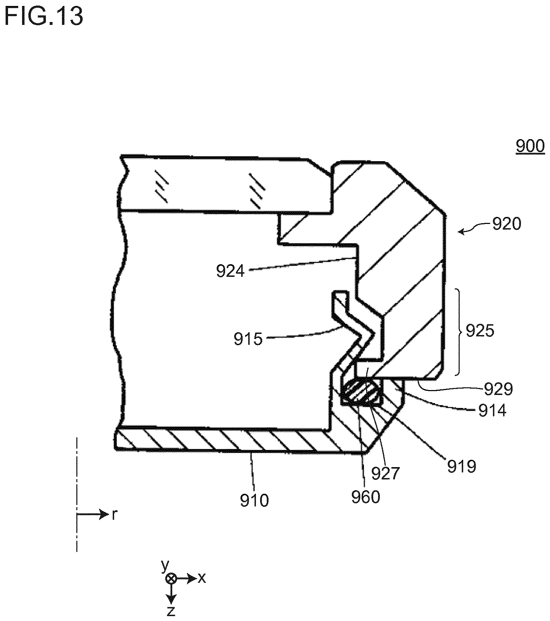

Fig. 13 is a view for describing a back cover mounting structure 900 of a watch as a background technique. Description of Embodiments

Hereinafter, a blood pump according to the present invention is described based on embodiments shown in drawings.

### [Embodiment 1]

### 1. Configuration of blood pump 100 according to embodiment 1

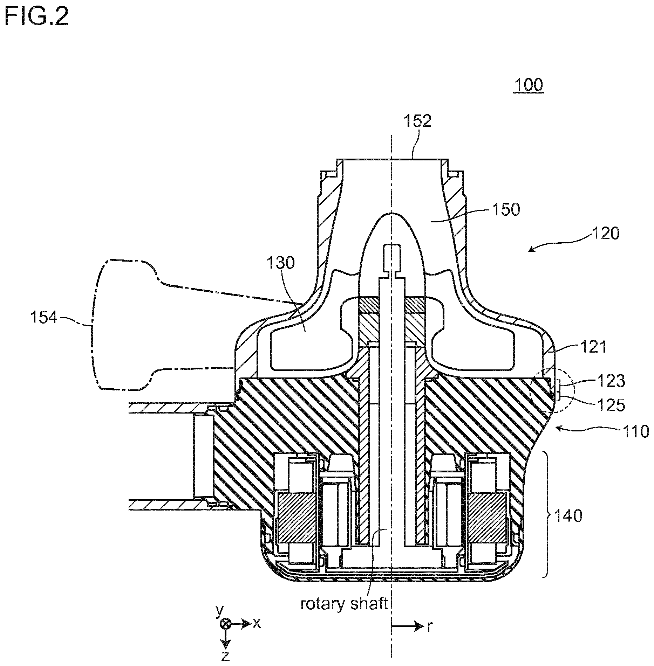

Fig. 1 is a perspective view of the blood pump 100 showing a state before a casing 120 is fitted on a base body 110. Fig. 2 is a cross-sectional view of the blood pump 100 showing a state after the casing 120 is fitted on the base body 110 where a cross section of the blood pump 100 taken along an xz plane shown in Fig. 1 is viewed along a y direction.

To roughly describe the overall structure of the blood pump 100 according to the embodiment 1, the blood pump 100 includes: a base body 110; a casing 120 fitted on the base body 110; a blood supply mechanism 130 housed in a pump chamber 150 surrounded by the base body 110 and the casing 120; and a drive element 140 mounted on the base body 110 and supplying energy to the blood supply mechanism 130. The blood pump 100 has a function of allowing blood to flow into the pump chamber 150 and to flow out from the pump chamber 150 by the blood supply mechanism 130 and supplying blood into the inside of the body of a user (see Fig. 1 and Fig. 2).

In the description made hereinafter, assume a direction that the casing 120 is fitted on the base body 110 by sliding as a z direction, a direction perpendicular to the z direction as an x direction, a direction perpendicular to the z direction and the x direction respectively as a y direction, a direction directed from a center portion of the base body 110 to the outside of the base body 110 when an xy plane is viewed in a plan view along the z direction as an r direction. Further, to facilitate the understanding of the blood pump 100, the z direction is referred to as "down", a -z direction is referred to as "up", a -r direction is referred to as "inside", the r direction is referred to as "radial direction" or "outside". With respect to the casing, a pump chamber side of the casing is referred to as "inside", a side opposite to the pump chamber is referred to as "outside", and a direction parallel to the xy plane is referred to as "horizontal" or the like.

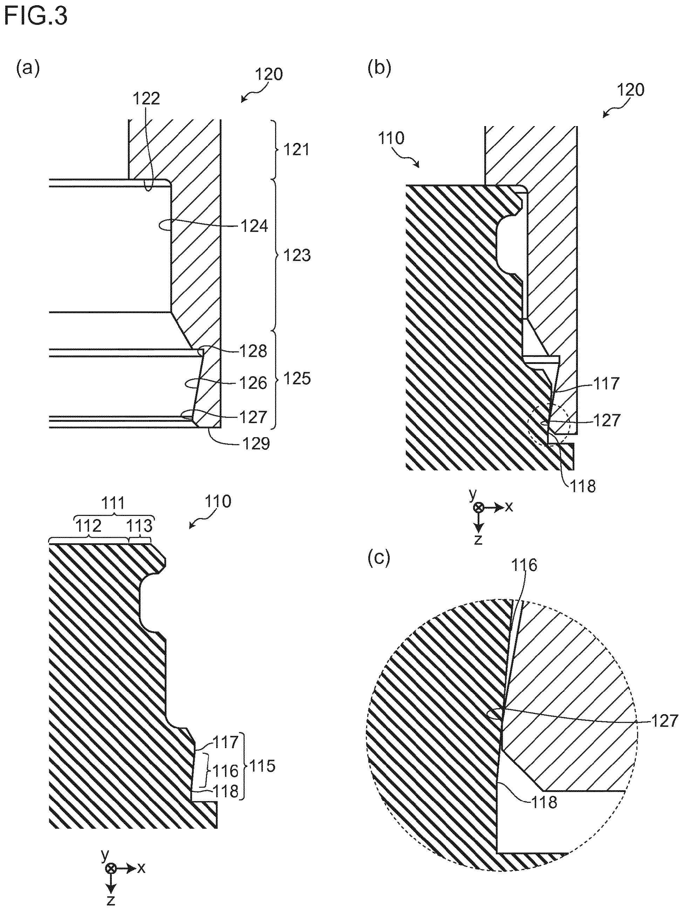

Fig. 3(a) and Fig. 3(b) are views showing an engaging portion surrounded by a dotted line in the cross-sectional view of Fig. 2 in an enlarged manner. Fig. 3 (a) is a cross-sectional view showing the base body 110 and the casing 120 in a state before the casing 120 is fitted on the base body 110, Fig. 3(b) is a cross-sectional view showing the base body 110 and the casing 120 in a state after the casing 120 is fitted on the base body 110, and Fig. 3(c) is a view of a portion surrounded by a dotted line in Fig. 3(b) in an enlarged manner.

As shown in Fig. 3(a) to Fig. 3(c), the base body 110 has an approximately horizontal surface 111 which is disposed in a -z direction side (upper side) and includes a blood contact surface 112 which faces the pump chamber 150 and a first contact surface 113 which is brought into contact with the casing 120. The blood contact surface 112 is a surface with which blood of a user is brought into contact, and defines the pump chamber 150 together with the casing body 121. The first contact surface is a surface which is brought into contact with a second contact surface 122 of the casing body 121 and receives an action of a pressing force (described later) from the second contact surface 122. These blood contact surface 112 and the first contact surface 113 are continuously formed, and form an approximately horizontal surface 111 as a whole.

Further, the base body 110 has a first engaging portion 115 which is formed on an r direction side and includes a first tapered portion 116 inclined toward the -r direction (inner side) as the first tapered portion 116 extends from an outer end portion 117 in the z direction (down) . The outer end portion 117 of the first tapered portion 116 protrudes most in the r direction side (outer side), and a terminal end portion 118 of the first tapered portion 116 is disposed on the most -r direction side (inner side).

The base body 110 may be formed using any material provided that the base body 110 forms a part of the blood pump 100 according to the present invention, and the base body 110 may be formed using the same material as the material for forming the casing 120. However, the base body 110 (particularly in the vicinity of the first engaging portion 115 and in the vicinity of the first contact surface 113) is made of a material which is sufficiently hard and can prevent bending to an extent that the base body 110 is not deformed even when a large force is applied from a second engaging portion 125 and the second contact surface 122 of the casing 120 described later. That is, the base body 110 (particularly in the vicinity of the first engaging portion 115 and in the vicinity of the first contact surface 113) has sufficient rigidity which allows the base body 110 to be considered as a rigid body.

On the other hand, as shown in Fig. 3(a), the casing 120 has: the casing body 121 where the second contact surface 122 is formed at a position which corresponds to the first contact surface 113; the second engaging portion 125 formed at an edge side of the casing 120 and including a second tapered portion 126 inclined toward the r direction (radial direction) as the second tapered portion 126 extends from an inner end portion 127 in the -z direction (up) on an inner wall of the casing 120; and a casing intermediate portion 123 positioned between the casing body 121 and the second engaging portion 125. Symbol 124 indicates an intermediate portion inner wall.

An inlet port 152 through which blood flows into the blood pump 100 and an outlet port 154 through which blood flows out from the blood pump 100 are formed on the casing body 121. An inner wall of the casing body 121 defines the pump chamber 150 (see together with Fig. 1 and Fig. 2). In the embodiment 1, the inlet port 152 and the outlet port 154 are formed separately from each other. However, the configuration may be adopted where an inlet port and an outlet port form a common port by designing such that a valve or the like is used outside the pump chamber 150.

The second contact surface 122 of the casing body 121 is a surface where a force directed in the z direction (down) which is generated in the second engaging portion 125 acts on the first contact surface 113 of the base body 110 as a pressing force by way of the casing intermediate portion 123.

As viewed in cross section shown in Fig. 3(a), one end (upper side) of the casing intermediate portion 123 is continuously formed with the casing body 121, and the other end (lower side) of the casing intermediate portion 123 is continuously formed with the second engaging portion 125.

The second tapered portion 126 which forms the second engaging portion 125 is formed in a tapered shape inclined toward the r direction (radial direction) as the second tapered portion 126 extends in the -z direction (up) from the inner end portion 127 to a neck portion 128. The inner end portion 127 of the second tapered portion 126 projects most toward the -r direction side (inner side).

The blood supply mechanism 130 is disposed in the inside of the pump chamber 150 surrounded by the base body 110 and the casing 120, and allows blood to flow into the pump chamber 150 and to flow out from the pump chamber 150 thus supplying blood to the inside of the body of a user using a movable mechanism which supplies blood (see Fig. 1 and Fig. 2).

The drive element 140 is mounted on the base body 110 and supplies energy for allowing the blood supply mechanism 130 to perform the supply of blood to the blood supply mechanism 130.

In the embodiment 1, an impeller is adopted as the blood supply mechanism 130, and a motor for driving the impeller is adopted as the drive element 140. The impeller easily exhibits a blood supply function, and a control technique which exhibits linearity, responsiveness and the like is relatively established with respect to the motor so that the motor can relatively easily perform a control in accordance with a purpose . This is because, by driving the impeller using such a motor, the blood supply mechanism 130 can exhibit an intended blood supply function efficiently, a blood pump having high performance and high accuracy can be provided.

In the embodiment 1, the motor is mounted on the base body 110 such that a stator part and a rotor part of the motor are housed in the base body 110 on the -z direction side as viewed from an approximately horizontal surface 111 of the base body 110. A rotary shaft of the motor projects from a base body 110 side toward the z direction side. The impeller is joined to this projecting rotary shaft (see Fig. 1 and Fig. 2).

In the blood pump 100 according to the embodiment 1, the second engaging portion 125 of the casing 120 engages with the first engaging portion 115 of the base body 110 in a state where the second engaging portion 125 is elastically deformed, and the second contact surface 122 of the casing 120 is pressed so as to be brought into contact with the first contact surface 113 of the base body 110 (see Fig. 3(a) to Fig. 3(c)).

In this embodiment, "pressed" does not mean a state where the second contact surface 122 simply butts against the first contact surface 113 but means a state where a pressing force is constantly applied to the second contact surface 122 and the first contact surface 113.

The first contact surface 113 and the second contact surface 122 are strongly pressed to each other by a pressing force while being brought into contact with each other and hence, blood in the pump chamber 150 is prevented from intruding between the first contact surface 113 and the second contact surface 122.

In this embodiment, "butting" is also referred to as "wholly brought into contact with each other" or the like. In a state where, for example, when a force is applied to a unit in the z direction, the unit and a counter unit which opposedly faces the unit are brought into contact with each other at a portion (or at a surface or the like), and a force in the z direction is applied to the whole surface of the counter unit at the portion (at the surface), such a portion (surface) is referred to as a "butting" portion (surface).

Hereinafter, further detailed portions of the blood pump 100 according to the embodiment 1 are described.

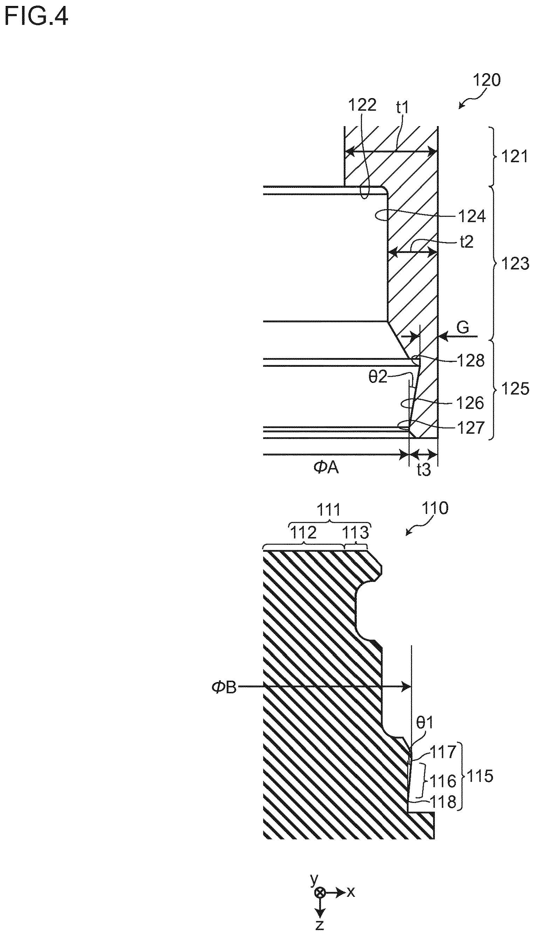

In the blood pump 100 according to the embodiment 1, assuming an inner diameter of the inner end portion 127 of the second tapered portion 126 of the casing 120 as φA and an outer diameter of the outer end portion 117 of the first tapered portion 116 of the base body 110 as φB before the casing 120 is fitted on the base body 110, a relationship of φA<φB is established, and the casing 120 is fitted on the base body 110 in a state that the inner end portion 127 of the second tapered portion 126 is positioned at a portion of the first tapered portion 116 shifted in the z direction side from the outer end portion 117, the portion being retracted from a terminal end portion 118 of the first engaging portion 115 formed on a side opposite to the outer end portion 117 (see Fig. 3(a) to Fig. 3(c) and Fig. 4).

That is, the inner end portion 127 of the second tapered portion 126 on the casing 120 side is in a state where the inner end portion 127 is disposed at a position on the z direction (down) side getting over the outer end portion 117 of the first tapered portion 116 on the base body 110 side. During a period before such a state is brought about, at the time of performing assembling (fitting engagement), when the inner end portion 127 of the casing 120 gets over the outer end portion 117 of the base body 110, an edge side of the casing 120 is expanded and is deformed at maximum in the r direction (outer side) thus generating a resilient force. Also when the inner end portion 127 is at a portion of the base body 110 where the inner end portion 127 is shifted in the z direction side from the outer end portion 117, an elastic force is maintained. Due to an effect of the first tapered portion 116, the elastic force is converted into a force which presses the second engaging portion 125 (and/or the whole casing) of the casing 120 in the z direction (down) by way of the inner end portion 127 and hence, it is possible to apply a large pressing force from the second contact surface 122 on the casing 120 side to the first contact surface 113 on the base body 110 side. Further, the inner end portion 127 is positioned at the portion shifted from the outer end portion 117 in the z direction side and hence, it is difficult for the inner end portion of the casing 120 to move in the -z direction (up). Accordingly, the above-mentioned pressing force can be "constantly" applied.

Further, in the blood pump 100 according to the embodiment 1, the casing lower end portion 129 on an edge side of the casing 120 is not brought into contact with the base body 110 in a direction along the z direction (see Fig. 3(a) to Fig. 3(c)).

Due to such a configuration, the casing lower end portion 129 is not brought into contact with the base body 110 and is brought into a so called free state. For example, even if the casing is extended from an initial state (or even if the base body is shrunk from an initial state) due to a change in temperature or the like, since an elongation margin is ensured in front of the casing lower end portion 129, there is no possibility that the casing lower end portion 129 butts against the base body 110 so that the extension of the casing is restricted whereby the above-mentioned pressing force is decreased. Accordingly, a large pressing force can be constantly applied from the second contact surface 122 on the casing 120 side to the first contact surface 113 on the base body 110 side.

Further, in the blood pump 100 according to the embodiment 1, it is preferable that, as viewed in a cross section of the blood pump 100 taken along an xz plane, assuming an angle made by a profile of an inclined surface of the first tapered portion 116 and the z direction as θ1 and an angle made by a profile of an inclined surface of the second tapered portion 126 and the z direction as θ2, a relationship of θ1<θ2 be established (see Fig. 3(a) to Fig. 3(c) and Fig. 4).

With such a configuration, due to the relationship of θ1<θ2, the inner end portion 127 of the casing 120 is brought into contact with the first tapered portion 116 of the base body 110 and hence, as described previously, due to the effect of the first tapered portion 116, a large pressing force can be constantly applied from the second contact surface 122 on the casing 120 side to the first contact surface 113 on the base body 110 side by way of the inner end portion 127.

For a reference purpose, even when θ1 and θ2 are substantially equal to each other, provided that the first engaging portion 115 and the second engaging portion 125 are formed with high accuracy and in a state where the casing lower end portion 129 is not brought into contact with the base body 110 in the direction along the z direction, the above-mentioned pressing force based on the corresponding resilient force can be generated.

Further, in the blood pump 100 according to the embodiment 1, it is preferable that, as viewed in a cross section of the blood pump 100 taken along an xz plane, assuming a thickness of the casing body 121 in a direction perpendicular to a tangent plane on an outer side of the casing body 121 as t1, a thickness of the casing intermediate portion 123 in a direction perpendicular to a tangent plane of an outer side of the casing intermediate portion 123 as t2, and a thickness of the second engaging portion 125 in a direction perpendicular to a tangent plane of an outer side of the second engaging portion 125 as t3, a relationship of t1>t2>t3 be established and, with respect to an inclined surface which forms the second tapered portion 126 of the second engaging portion 125, at a portion on an end side in the -z direction which is a side opposite to the inner end portion 127 (neck portion 128), the casing 120 have a smallest thickness (see Fig. 3(a) to Fig. 3(c) and Fig. 4). Further, at the time of fitting, it is preferable that the casing intermediate portion 123 be elastically deformed although an amount of elastic deformation of the casing intermediate portion 123 is smaller than an amount of elastic deformation of the second engaging portion 125.

With such a configuration, the thickness of the casing 120 is gradually decreased in a stepwise manner from the casing body 121 to the second engaging portion 125. Accordingly, although a deformation of the casing intermediate portion 123 is smaller than a deformation of the second engaging portion 125, the casing intermediate portion 123 is also elastically deformed to some extent so that it is possible to efficiently impart a strong elastic force to the casing 120 as a whole similar to a fishing rod. The casing 120 may have a neck portion 128 where a thickness of the casing 120 is temporarily smaller than the other portion of the casing 120 thus making a "neck" in the midst of the casing 120 from the casing body 121 to the second engaging portion 125. By forming the neck portion 128, resilient force can be further efficiently generated.

In the blood pump 100 according to the embodiment 1, in a state where the first engaging portion 115 and the second engaging portion 125 are cut along an xy plane and are viewed in a plan view along the z direction, the first engaging portion 115 and the second engaging portion 125 are formed in a circular shape respectively. In this embodiment, "circular shape" means that the shape has no corners, and an elliptical shape, a true circular shape and the like are named as "circular shape", for example.

In this manner, by forming the first engaging portion 115 and the second engaging portion 125 in a circular shape, when the casing 120 is fitted on the base body 110, an elastic force is uniformly generated as a whole so that a pressing force is also uniformly generated whereby it is possible to provide the blood pump 100 having the stable fitting engagement structure.

In the blood pump 100 according to the embodiment 1, the second engaging portion 125 of the casing 120 may have the structure where "slits" are formed along an outer periphery of the second engaging portion 125. However, it is preferable that the second engaging portion 125 have the continuous ring-shaped structure (approximately circular cylindrical shape) having no "slits".

With such a structure, when the inner end portion 127 of the casing 120 gets over the outer end portion 117 of the base body 110, the second engaging portion 125 can generate a larger elastic force than the second engaging portion having "slits". With such a structure, even when thicknesses of the second engaging portion 125, the casing intermediate portion 123 and the like of the casing 120 are set small (thin), the casing 120 can ensure a sufficient elastic force. Such a structure also contributes to the miniaturization and the reduction of weight of the blood pump 100.

In the blood pump 100 according to the embodiment 1, a mirror finish is applied to the approximately horizontal surface 111 of the base body 110. For example, it is preferable that a value of Ra (arithmetic average roughness) be approximately 1.0 or less, and it is more preferable that a value of Ra (arithmetic average roughness) be approximately 0.2 or less.

With such a configuration, the first contact surface 113 which forms a part of the approximately horizontal surface 111 is brought into contact with the second contact surface 122 with a smoother surface and hence, it is possible to make the formation of a gap between the first contact surface 113 and the second contact surface 122 more difficult. Further, a mirror finish is also applied to the blood contact surface 112 which forms a part of the approximately horizontal surface 111 of the base body 110 and hence, blood minimally adheres to the surface whereby the generation of a thrombus by stagnation of blood can be further effectively prevented.

"Approximately horizontal surface 111" means a surface which becomes horizontal when the blood pump 100 is placed in a usual state. However, "approximately horizontal surface 111" also includes a case where "horizontal" has slight deviation. Further, even when "approximately horizontal surface 111" is not horizontal (vertical or the like) in view of designing or a use state, in this embodiment, such a state is also defined as "horizontal surface" or "approximately horizontal surface" for the sake of convenience. Further, "approximately horizontal surface 111" also includes a case where the plane 111 is not formed of a completely flat surface. For example, the blood contact surface 112 may be plane which draws a slight curve.

The casing 120 may be formed using any material which can be used for forming the blood pump 100 according to the embodiment 1. For example, it is possible to adopt materials such as pure titanium (F67, grade 2 or the like stipulated in ASTM standard, second type stipulated in JIS standard or the like), a titanium alloy, stainless steel (SUS or the like), or other various alloys. Further, in forming the second engaging portion, it is possible to use a material belonging to a resin provided that the second engaging portion is made of such a material which can generate an elastic force thus generating a required pressing force.

However, in the embodiment 1, it is preferable that the casing 120 be made of a material which contains titanium as a main component. (1) Titanium is a material whose biocompatibility is confirmed and is permitted as a medical-use material which can be incorporated into a human body. (2) Titanium has a sufficient tensile strength, a sufficient yield strength, and a high specific strength. Accordingly, even when a thickness of the casing made of titanium is made small, the casing exhibits a high strength and is light-weighted, and has appropriate elasticity. Titanium has physical properties suitable for forming the casing according to the present invention. From various viewpoints including the above-mentioned viewpoints, with the use of titanium as a material for forming the casing, it is possible to provide a blood pump for a highly rational auxiliary artificial heart.

In the blood pump 100 according to the embodiment 1, defining a diameter of the inner end portion 127 of the second engaging portion 125 of the casing 120 as ϕA, a diameter of the outer end portion 117 of the first engaging portion 115 of the base body 110 as ϕB, a thickness of the casing 120 in the vicinity of the neck portion 128 of the second engaging portion 125 in the r direction as G, a thickness of the casing 120 in the vicinity of the inner end portion 127 of the second engaging portion 125 in the r direction as t3, and θ1 and θ2 as defined above, it is preferable that (i) a difference between ϕA and ϕB be a difference within a range of approximately 0.1% to 1.0% of ϕB, and (ii) when a size of ϕA and ϕB is approximately 35 mm to 60 mm, G fall within a range of 0.15 mm to 0.40 mm, and t3 be approximately twice as large as G, and fall within a range of 0.35 mm to 0.8 mm, for example.

Further, it is preferable that θ1 fall within a range of 2° to 10° and θ2 fall within a range of 4° to 20°. It is preferable that θ2 be approximately twice as large as θ1 (see Fig. 4).

Further, in the blood pump 100 according to the embodiment 1, (i) when an inner pressure of the pump chamber 150 is a pressure which falls within a range of 0 mmHg to 200 mmHg, the second contact surface 122 does not float from the first contact surface 113 (generates no gap). (ii) It is preferable that, when the inner pressure of the pump chamber 150 is a pressure which falls within a range of 0 mmHg to 500 mmHg, the second contact surface 122 do not float from the first contact surface 113 (generates no gap). (iii) It is further preferable that, when the inner pressure of the pump chamber 150 is a pressure which falls within a range of 0 mmHg to 1500 mmHg, the second contact surface 122 do not float from the first contact surface 113 (generates no gap).

### 2. Manner of operation and advantageous effects of blood pump 100 according to the embodiment 1

### (1) Manner of operation

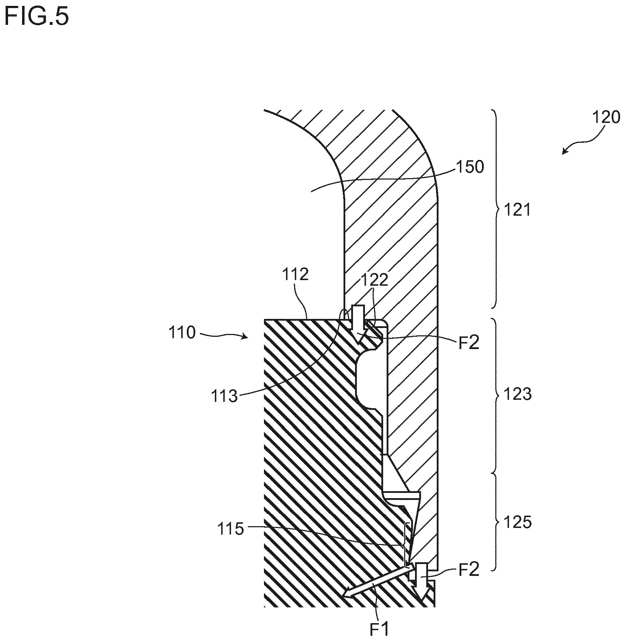

Fig. 5 is a view for describing a manner in which a pressing force F2 which acts from the second contact surface 122 to the first contact surface 113 is generated in the blood pump 100 according to the embodiment 1.

In the blood pump 100 according to the embodiment 1, the first engaging portion 115 having the first tapered portion 116 and the second engaging portion 125 having the second tapered portion 126 engage with each other (see Fig. 4 and Fig. 5).

Before such a state is brought about, in an assembling step of the blood pump 100, the casing 120 is fitted on the base body 110 by press fitting from an extremely tight state. In the casing 120 fitted on the base body 110, an edge side of the casing 120 is elastically deformed by being expanded in the r direction side (outer side) as a whole compared to a state before fitting. Due to such elastic deformation, an elastic force F1 (a force which fastens the base body 110) which intends to return in the -r direction (inner side) is generated. Particularly, an extremely large elastic force F1 acts on the first tapered portion 116 around an area in the vicinity of the inner end portion 127 of the second engaging portion 125. On the other hand, the base body 110 side including the first tapered portion 116 is regarded as a rigid body as described above. Accordingly, when the elastic force F1 acts, reversely, a resistance force perpendicular to the second engaging portion 125 is generated in a direction perpendicular to a contact surface of the first tapered portion 116. Along with the generation of the resistance force, due to an effect of the inclined surface by the first tapered portion 116, a force F2 which is a component in the z direction (downward direction) of the perpendicular resistance force is also generated. This force F2 is a force which presses down the whole casing 120 in the z direction (down) . In an interlocking manner with such a pressing operation, a large pressing force F2 is applied from the second contact surface 122 on the casing 120 side to the first contact surface 113 on the base body 110 side. Further, the inner end portion 127 is positioned at the portion shifted from the outer end portion 117 in the z direction side and hence, it is difficult for the inner end portion of the casing 120 to move in the -z direction (up). Accordingly, it is possible to "constantly" add a force to the above-mentioned pressing force (see Fig. 3 (a) to Fig. 3(c) together with Fig. 5).

The first contact surface 113 of the base body 110 functions as a surface which butts against the second contact surface 122 of the casing 120, and it is desirable that the base body 110 have no other portions which receive a force from the casing in the z direction (down).

### (2) Advantageous effects

As a result of the above-mentioned manner of operation (1), the second contact surface 122 of the casing 120 is constantly pressed so as to be brought into contact with the first contact surface 113 of the base body 110. Accordingly, the blood pump 100 according to the first embodiment can acquire at least the following advantageous effects (a) to (d).
(a) Even when a pressure in the pump chamber is increased, it is possible to prevent the formation of a gap between the second contact surface on the casing side which defines the pump chamber and the first contact surface on the base body side which defines the pump chamber (the second contact surface does not float from the first contact surface).
(b) By adopting the above-mentioned fitting engagement structure, it is unnecessary to use screws. Accordingly, while satisfying the above-mentioned required specification of the blood pump, portions irrelevant to a basic function (blood supply function) of the blood pump can be made as small as possible and hence, the blood pump can be formed with a margin MG1 which is far smaller than the screw fastening margin MG2 in the conventional blood pump. As a result, it is possible to provide the useful blood pump having a small diameter and a small volume compared to conventional blood pumps.
(c) It is possible to provide the useful blood pump which can achieve both of the above-mentioned advantageous effects (a) and (b).
(d) Due to the above-mentioned advantageous effect, as a matter of course, it is possible to provide the blood pump which can prevent a leakage of blood to the outside and can prevent the intrusion of a bodily fluid or the like from the outside of the blood pump into the inside of the blood pump.

### 3. Auxiliary artificial heart system 300 using the blood pump 100

It is needless to say that the blood pump 100 according to the embodiment 1 may be mounted outside the body of a user. However, by using the blood pump 100 according to the embodiment 1 in the form of an auxiliary artificial heart by embedding the blood pump 100 in the inside of the body, the blood pump 100 can further enjoy advantages from a view point of miniaturization and the reduction of weight.

When the blood pump is embedded in the inside of the body, the blood pump is embedded in a chest of a user (patient) having a limited thickness. Accordingly, with the use of the blood pump according to the present invention which has a small diameter and a small volume compared to conventional blood pumps while satisfying fundamental required specifications, the number of people who can use a blood pump can be increased. It is also possible to satisfy a demand in a medical field more preferably.

In this specification, the expression that the blood pump is "embedded" in the body is used. However, besides such an expression, it is possible to use the expression that the blood pump is "implanted" in the body.

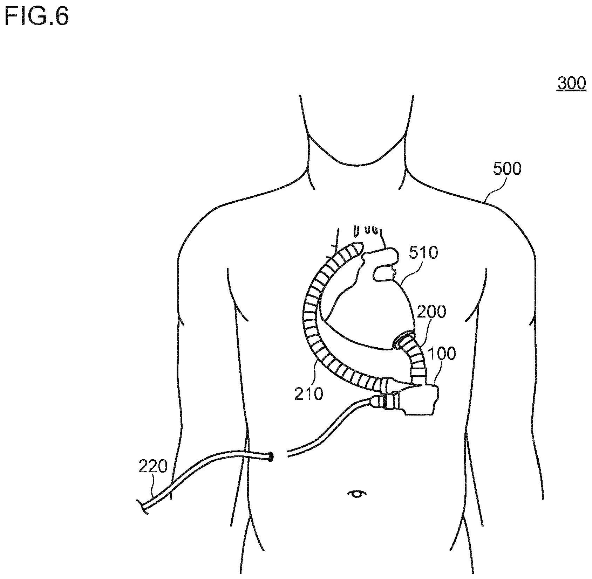

Fig. 6 is a schematic view for describing the auxiliary artificial heart system 300 where the blood pump 100 according to the embodiment 1 is embedded into the body.

For example, as shown in Fig. 6, the auxiliary artificial heart system 300 includes: the blood pump 100 embedded in the body of a user; an artificial blood vessel 200 which connects the blood pump 100 and a left ventricle (not shown in the drawing) in the actual heart 510 of the user to each other; an artificial blood vessel 210 provided for returning blood from the blood pump 100 to the inside of the body of the user; a controller (not shown in the drawing) disposed outside the body of the user for controlling an operation of the blood pump 100; a cable 220 connecting the controller and the blood pump 100 to each other and the like.

As has been described heretofore, with the use of the blood pump 100 according to the embodiment 1 which has a small diameter and a small volume compared to the conventional blood pump while satisfying required specifications, for example, it is possible to embed the blood pump 100 into the inside of the body of a person (patient) having a small physical build such as a child and hence, it is expected that the number of people who can use a blood pump is remarkably increased.

### [Embodiment 2]

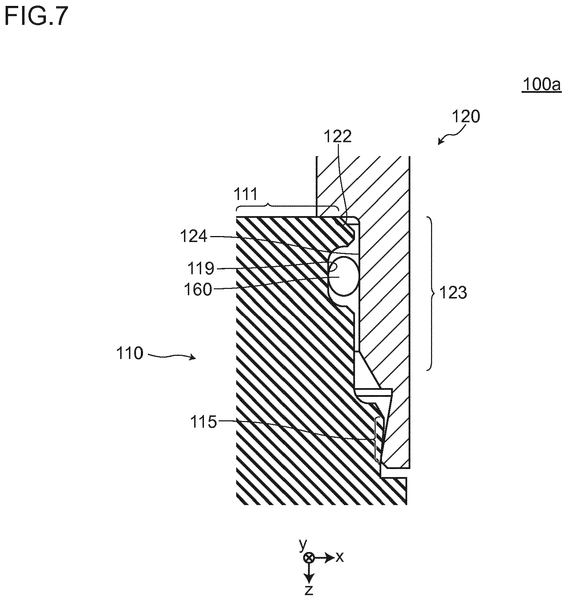

Next, a blood pump 100a according to an embodiment 2 is described with reference to Fig. 7.

Fig. 7 is a view for describing a main part of the blood pump 100a according to the embodiment 2, and is a view showing an engaging portion surrounded by a dotted line in the cross-sectional view of Fig. 2 in an enlarged manner. Parts having substantially the same configuration as corresponding parts of the embodiment 1 are given the same numerals.

The blood pump 100a according to the embodiment 2 has basically substantially the same configuration as the blood pump 100 according to the embodiment 1. However, the blood pump 100a according to the embodiment 2 differs from the blood pump 100 according to the embodiment 1 with respect to a point that the configuration relating to a packing 160 is added. That is, as shown in Fig. 7, in the blood pump 100a according to the embodiment 2, a base body 110 has a packing groove 119 in which the packing 160 is disposed at a position on a more -z direction side (upper side) of a first engaging portion 115, a casing 120 has an intermediate portion inner wall 124 at a position of a casing intermediate portion 123, and the packing 160 is disposed such that the packing 160 is sandwiched between the packing groove 119 and the intermediate portion inner wall 124.

In this manner, the blood pump 100a according to the embodiment 2 differs from the blood pump 100 according to the embodiment 1 with respect to the point that the configuration relating to the packing 160 is added. However, according to the blood pump 100a of the embodiment 2, besides a contact portion which seals a pump chamber 150 by a first contact surface 113 and a second contact surface 122, the packing 160 is further disposed outside the contact portion and hence, a leakage of blood to the outside of the blood pump can be blocked by double sealing, and the intrusion of a bodily fluid or the like from the outside of the blood pump into the inside of the blood pump can be blocked. The packing 160 is collapsed in the -r direction or the r direction due to a resilient force of the casing intermediate portion 123 and hence, it is unnecessary to increase a diameter of the blood pump.

In the blood pump 100a according to the embodiment 2, when the blood pump 100a is viewed in a plan view along the z direction, it is preferable that the packing groove 119 formed on the base body 110 overlap with an approximately horizontal surface 111 of the base body 110, and the second contact surface 122 of the casing 120 overlap with at least a portion of the packing 160.

According to the blood pump 110a having such a configuration, the packing 160 is assembled into the blood pump 100a in the form that at least a portion of a thickness of the packing 160 in the r direction is absorbed in a region of the approximately horizontal surface 111 and the second contact surface 122. Accordingly, the packing can be added without particularly increasing a diameter of the blood pump and hence, it is possible to provide a blood pump having a small diameter and a small volume.

The blood pump 100a according to the embodiment 2 has substantially the same configuration as the blood pump 100 according to the embodiment 1 except for the configuration relating to the packing 160. Accordingly, the blood pump 100a according to the embodiment 2 directly acquires the corresponding advantageous effects found amongst all advantageous effects which the blood pump 100 according to the embodiment 1 acquires.

### [Embodiment 3]

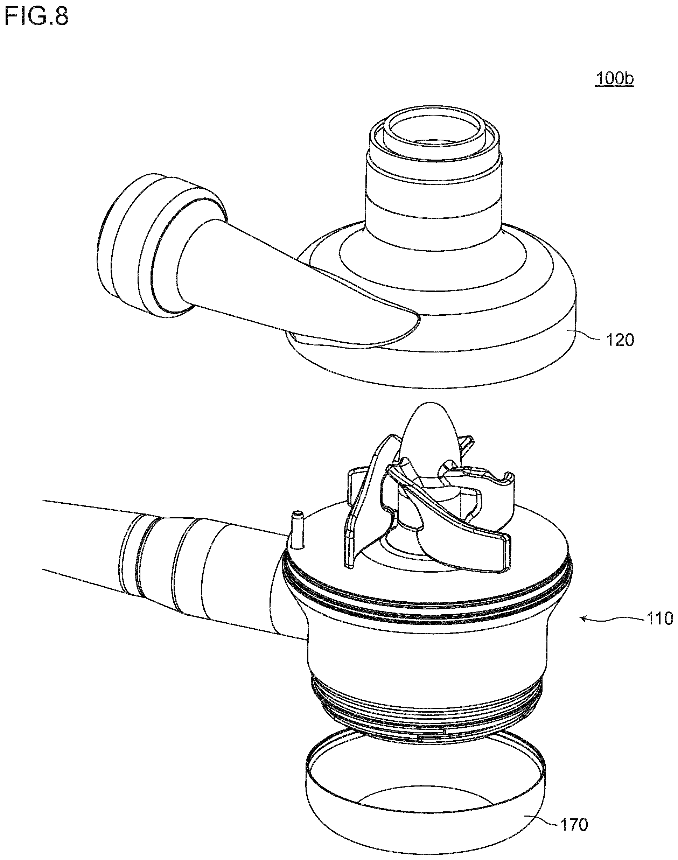

Next, a blood pump 100b according to an embodiment 3 is described with reference to Fig. 8.

Fig. 8 is a view for describing the blood pump 100b according to the embodiment 3, and is a perspective view showing a state before a casing 120 and another casing 170 are respectively fitted on a base body 110.

The blood pump 100b according to the embodiment 3 has basically substantially the same configuration as the blood pump 100 according to the embodiment 1 and the blood pump 100a according to the embodiment 2. However, the blood pump 100b according to the embodiment 3 differs from the blood pump 100 according to the embodiment 1 and the blood pump 100a according to the embodiment 2 with respect to a point that another casing 170 is fitted on the base body 110 on a z direction side (bottom surface side) of the base body 110.

That is, as shown in Fig. 8, in the blood pump 100b according to the embodiment 3, the base body 110 has a housing chamber (not shown in the drawing) which opens on a z direction side (bottom surface side) and houses a drive element (a rotor part of a motor) and the like therein. That is, the drive element (the rotor part of the motor) is housed in the housing chamber. Further, by fitting another casing 170 on the base body 110 on the z direction side (bottom surface side), the housing chamber forms a casing. The fitting engagement structure between another casing 170 and the base body 110 has substantially the same structure as the fitting engagement structure between the casing 120 and the base body 110 in the embodiment 1. That is, another first engaging portion substantially equal to the first engaging portion 115 is formed on the base body 110 on the z direction side (bottom surface side), and another second engaging portion substantially equal to the second engaging portion 125 is formed on an edge side of another casing 170. Another first engaging portion and another second engaging portion are engaged with each other by fitting engagement using the structure substantially equal to the structure used in the embodiment 1.

In this manner, the blood pump 100b according to the embodiment 3 differs from the blood pump 100 according to the embodiment 1 and the blood pump 100a according to the embodiment 2 with respect to the point that another casing 170 is fitted on the base body 110 on the z direction side (bottom surface side) of the base body 110. However, in the blood pump 100b according to the embodiment 3, another casing 170 is fitted on the base body 110 from the z direction side (bottom surface side) using the structure substantially equal to the structure used in the embodiment 1. Accordingly, even in the case where it is necessary to provide the above-mentioned housing chamber, the housing chamber can be closed by another casing 170 without ensuring a large margin (a fastening margin or the like) and hence, it is possible to provide a blood pump having a small diameter and a small volume.

The blood pump 100b according to the embodiment 3 has substantially the same configuration as the blood pump 100 according to the embodiment 1 and the blood pump 100a according to the embodiment 2 except for a point that another casing 170 is fitted on the base body 110 on the z direction side (bottom surface side) of the base body 110. Accordingly, the blood pump 100b according to the embodiment 3 directly acquires the corresponding advantageous effects found amongst all advantageous effects which the blood pump 100 according to the embodiment 1 and the blood pump 100a according to the embodiment 2 acquire.

### [Example]

An example of the blood pump according to the present invention is described hereinafter.

### (1) Blood pumps according to example

The blood pump according to the embodiment 2 and the blood pump according to the embodiment 3 were manufactured as models and were used as blood pumps according to examples.

That is, the blood pump according to the example was manufactured such that the blood pump also includes, in addition to the fitting engagement structure formed of a base body 110 and a casing 120 according to the embodiment 1, a packing 160 and another casing 170 (back cover) 170. An impeller was adopted as a blood supply mechanism 130, a motor was adopted as a drive element 140, and pure titanium (F67, grade 2 stipulated in ASTM standard) was adopted as a material for forming the base body 110 and the casing 120.

### (2) Blood pump according to comparison example

As a comparison example 1, a blood pump which adopts the back cover mounting structure (background art) of a watch which is used in general was manufactured as a model. Further, as a comparison example 2, the conventional blood pump 800 (see Fig. 11 and Fig. 12) was manufactured as a model.

With respect to the comparison example 1 and the comparison example 2, as the structure for joining a base body and a casing, the comparison example 1 adopts the structure which uses dowel portions, and the comparison example 2 adopts the structure which uses screws . With respect to the structures, sizes and the like of portions relating to basic functions (the structures, sizes and the like of an impeller, a pump chamber, a blood contact surface, an inlet port, an outlet port and the like) other than the above-mentioned structures, the comparison example 1 and the comparison example 2 basically adopt the same structures as the examples.

### (3) Advantageous effects obtained by blood pumps according to examples

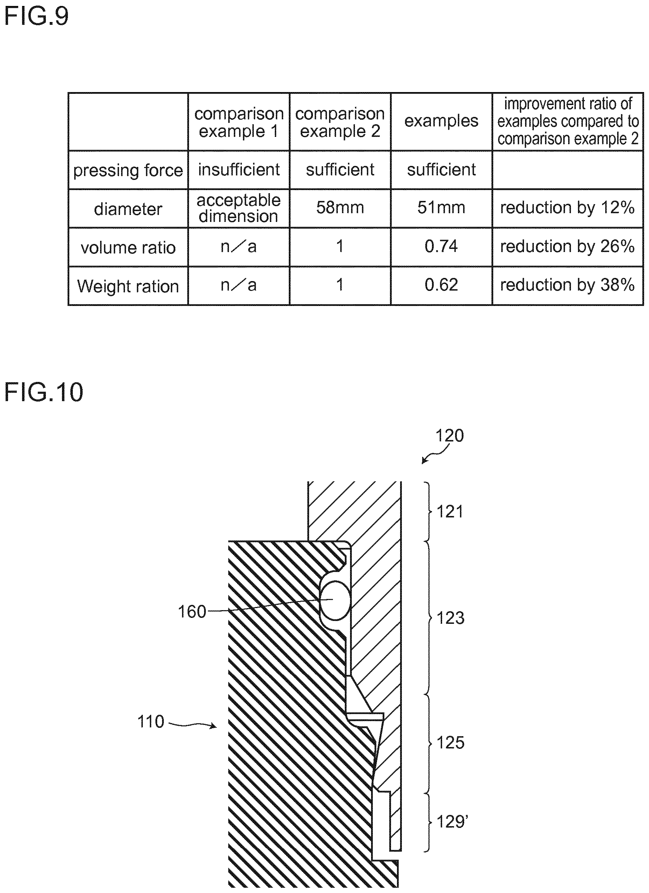

Fig. 9 is a view for describing an evaluation result of the blood pumps according to the examples.

As shown in Fig. 9, from a viewpoint of a pressing force of a second contact surface to a first contact surface, it was confirmed that the blood pump according to the comparison example 2 and the blood pump according to the example could acquire a sufficient performance. From a viewpoint of miniaturization and reduction of weight, it was confirmed that the blood pump according to the example can reduce a diameter of the whole blood pump by 12% compared to the blood pump according to the comparison example 2, can reduce a volume of the whole blood pump by 26% compared to the blood pump according to the comparison example 2, and can reduce a weight of the whole blood pump by 38% compared to the blood pump according to the comparison example 2.

From the above, it was confirmed that the blood pump according to the present invention has a small diameter and a small volume compared to the conventional blood pump while satisfying a required specification that it is possible to constantly apply a large pressing force from a contact surface on a casing side to a contact surface on a base body side so that, even when a pressure in a pump chamber is increased, a gap is not formed between the contact surface on the casing side which defines the pump chamber and the contact surface on the base body side which defines the pump chamber.

Although the present invention has been described based on the above-mentioned embodiments heretofore, the present invention is not limited to the above-mentioned embodiments. The present invention can be carried out without departing from the gist of the present invention, for example, the following modifications are also conceivable.
(1) The numbers, the materials, the shapes, the positions, the sizes, the angles and the like of the constitutional elements described in the above-mentioned respective embodiments are provided only for an exemplifying purpose, and these can be changed within ranges where advantageous effects of the present invention are not impaired.
(2) In the above-mentioned respective embodiments, the end of the second engaging portion 125 on the most z direction side is also the end of the casing 120 on the most z direction side (casing lower end portion 129). However, the present invention is not limited to such a configuration. For example, as shown in Fig. 10, a structure may be adopted where a cover portion 129' which does not contribute to engagement is provided on a more z direction side than a portion of a second engaging portion 125, and the z direction side (lower end) of the cover portion 129' forms an end of the casing 120 on the most z direction side (casing lower end portion 129). By providing the cover portion 129', it is possible to protect a first engaging portion 115 and a second engaging portion 125 which generate a pressing force. However, it is desirable that the end of the cover portion 129' on the z direction side (casing lower end portion 129) be not brought into contact with the base body 110 in a direction along the z direction.

Further, in the case where a packing 160 is disposed, in Fig. 10, the packing 160 is disposed at a position corresponding to a casing intermediate portion 123. However, the arrangement of the packing 160 is not limited to such arrangement. For example, the packing 160 may be disposed at the position of the cover portion 129'.
(3) In the above-mentioned embodiment 2, the packing groove 119 and the intermediate portion inner wall 124 of the casing 120 which opposedly faces the packing groove 119 are respectively formed along the z direction, and are disposed parallel to each other (see Fig. 7). However, the present invention is not limited to such a configuration. For example, the packing groove 119 and the intermediate portion inner wall 124 may not be disposed parallel to each other, and the intermediate portion inner wall 124 may be formed in a tapered shape so as to be inclined in a -r direction side (inner side) as the intermediate portion inner wall 124 extends in a z direction (down) (not shown in the drawing). The packing 160 disposed between an intermediate portion inner wall 124' having such an inclination and the packing groove 119 generates an elastic force (repulsive force) when the packing 160 is sandwiched. Such an elastic force (repulsive force) pushes the intermediate portion inner wall 124' and the packing groove 119, and becomes an additional force which forms a part of a force which eventually pushes down the casing 120 in the z direction.

As an opposite case, a taper which acquires substantially the same advantageous effect may be formed on the packing groove 119 by changing a design of a packing groove 119 side.
(4) In the above-mentioned respective embodiments, the description has been made by estimating the case where the first contact surface 113 and the second contact surface 122 are disposed parallel to each other. However, the present invention is not limited to such a configuration. For example, the second contact surface 122 may not be formed parallel to a zy plane (that is, may not be formed horizontally), and may be inclined in the z direction (down) as the second contact surface 122 extends in the -r direction (inner side).

With such a configuration, although the second contact surface 122 which is brought into contact with the first contact surface 113 may have a smaller contact area than the above-mentioned respective embodiments, the above-mentioned pressing force F2 is applied with a small area and hence, a higher pressure can be applied to the first contact surface 113.
(5) In the above-mentioned respective embodiments, the casing intermediate portion 123 is disposed between the casing body 121 and the second engaging portion 125. However, the present invention is not limited to such a configuration. The present invention may adopt a structure where the casing intermediate portion 123 is omitted.
(6) In the above-mentioned respective embodiments, an impeller is adopted as the blood supply mechanism 130. However, the present invention is not limited to such a configuration. For example, a vaneless-type blood supply mechanism, a cylinder-type blood supply mechanism, a pulsation-type blood supply mechanism, a turbo-type blood supply mechanism, a volume-type blood supply mechanism, a flagellar movement-type blood supply mechanism or the like may be adopted.
(7) In the above-mentioned blood pumps according to the respective embodiments, the description has been made by estimation the case where the blood pump is used in a state where the blood pump is embedded in the body of a user. However, the present invention is not limited to such a configuration. The blood pump according to the present invention is usefully used even in the case where the blood pump is installed outside the body of a user. This is because it is necessary to carry the blood pump along with the movement of the user and hence, a demand for a small sized and light-weighted blood pump is still strong. Reference Signs List

110, 810: base body
100, 100a, 100b, 800: blood pump
111: approximately horizontal surface
112, 812: blood contact surface
113, 813: first contact surface
115: first engaging portion
116: first tapered portion
117: outer end portion
118: terminal end portion
119: packing groove
120, 820: casing
121: casing body
122, 822: second contact surface
123: casing intermediate portion
124, 124': intermediate portion inner wall
125: second engaging portion
126: second tapered portion
127: inner end portion
128: neck portion
129: casing lower end portion
129': cover portion
130, 830: blood supply mechanism
140, 840: drive element
150, 850: pump chamber
152: inlet port
154: outlet port
160, 960: packing
170: another casing
200, 210: artificial blood vessel
220: cable
300: auxiliary artificial heart system
510: actual heart
814: screw fastening margin
815: female screw
825: play hole
890: male screw
900: back cover mounting structure
927: second engaging convex portion

## Claims

1. A blood pump comprising:
a base body;
a casing fitted on the base body;
a blood supply mechanism housed in a pump chamber surrounded by the base body and the casing; and
a drive element mounted on the base body for supplying energy to the blood supply mechanism, wherein
the blood supply mechanism is configured to allow blood to flow into the pump chamber and to flow out from the pump chamber supplying blood into the inside of a body of a user by the blood supply mechanism, wherein
assuming a direction that the casing is fitted on the base body by sliding as a z direction, a direction perpendicular to the z direction as an x direction, a direction perpendicular to the z direction and the x direction respectively as a y direction, and a direction directed from a center portion of the base body to the outside of the base body when an xy plane is viewed in a plan view along the z direction as an r direction,
the base body has:
an approximately horizontal surface formed on a -z direction side, and including a blood contact surface which faces the pump chamber and a first contact surface which is brought into contact with the casing; and
a first engaging portion formed on an r direction side and including a first tapered portion inclined toward a -r direction as the first tapered portion extends from an outer end portion of the first engaging portion in the z direction, and
the casing has:
a casing body having a second contact surface at a position which corresponds to the first contact surface;
a second engaging portion formed at an edge side of the casing, and including a second tapered portion inclined toward the r direction as the second tapered portion extends from an inner end portion in the -z direction on an inner wall of the casing; and
a casing intermediate portion positioned between the casing body and the second engaging portion, wherein
the blood pump is configured such that the second engaging portion of the casing engages with the first engaging portion of the base body, and the second contact surface of the casing is pressed so as to be brought into contact with the first contact surface of the base body.

2. The blood pump according to claim 1, wherein
assuming an inner diameter of the inner end portion of the second tapered portion of the casing as φA and an outer diameter of the outer end portion of the first tapered portion of the base body as φB before the casing is fitted on the base body, a relationship of φA<φB is established, and
the casing is fitted on the base body in a state that the inner end portion of the second tapered portion is positioned at a portion of the first tapered portion shifted in a z direction side from the outer end portion, the portion being retracted from a terminal end portion of the first engaging portion formed on a side opposite to the outer end portion.

3. The blood pump according to claim 1 or 2, wherein a casing lower end portion which is disposed on an edge side of the casing is not brought into contact with the base body in a direction along the z direction.

4. The blood pump according to any one of claims 1 to 3, wherein as viewed in a cross section of the blood pump taken along an xz plane, assuming an angle made by a profile of an inclined surface of the first tapered portion and the z direction as θ1 and an angle made by a profile of an inclined surface of the second tapered portion and the z direction as θ2, a relationship of θ1<θ2 is established.

5. The blood pump according to any one of claims 1 to 4, wherein as viewed in a cross section of the blood pump taken along an xz plane,
assuming a thickness of the casing body in a direction perpendicular to a tangent plane on an outer side of the casing body as t1, a thickness of the casing intermediate portion in a direction perpendicular to a tangent plane of an outer side of the casing intermediate portion as t2, and a thickness of the second engaging portion in a direction perpendicular to a tangent plane of an outer side of the second engaging portion as t3, a relationship of t1>t2>t3 is established, and
with respect to an inclined surface which forms the second tapered portion of the second engaging portion, at a portion on an end of the inclined surface in the -z direction which is a side opposite to the inner end portion, the casing has a smallest thickness.

6. The blood pump according to any one of claims 1 to 5, wherein a mirror finish is applied to the approximately horizontal surface of the base body.

7. The blood pump according to any one of claims 1 to 6, wherein as viewed in a plan view of the first engaging portion and the second engaging portion taken along an xy plane respectively along the z direction, the first engaging portion and the second engaging portion have a circular shape.

8. The blood pump according to any one of claims 1 to 7, wherein the casing is made of a material which contains titanium as a main component.

9. The blood pump according to any one of claims 1 to 8, wherein
the base body has a packing groove in which a packing is disposed at a position on a more -z direction side of the first engaging portion,
the casing has an intermediate portion inner wall at a position of the casing intermediate portion, and
the packing is disposed such that the packing is sandwiched between the packing groove and the intermediate portion inner wall.

10. The blood pump according to claim 9, wherein
when the blood pump is viewed in a plan view along the z direction, the packing groove formed in the base body overlaps with the approximately horizontal surface of the base body, and the second contact surface of the casing overlaps with at least a portion of the packing.

11. The blood pump according to any one of claims 1 to 10, wherein the blood supply mechanism is an impeller, and the drive element is a motor for driving the impeller.

12. The blood pump according to any one of claims 1 to 11, wherein the blood pump is used in the form of an auxiliary artificial heart by embedding the blood pump in the inside of the body.
